## Europäisches Patentamt

## European Patent Office

## Office européen des brevets

(11) Veröffentlichungsnummer: **0 157 224**
A2

(12) # EUROPÄISCHE PATENTANMELDUNG

(21) Anmeldenummer: **85102671.6**

(51) Int. Cl.⁴: **A 61 M 5/31**

(22) Anmeldetag: **08.03.85**

(30) Priorität: **31.03.84 DE 8410069 U**

(43) Veröffentlichungstag der Anmeldung: **09.10.85**
Patentblatt 85/41

(84) Benannte Vertragsstaaten: **AT BE CH DE FR GB IT LI LU NL SE**

(71) Anmelder: **INTERMEDICAT GMBH, Gerliswilstrasse 74, CH-6020 Emmenbrücke (CH)**

(72) Erfinder: **Rycyk, Manfred, Herkulesstrasse 19, D-3501 Körle (DE)**
Erfinder: **Berent, Arved, Quillerstrasse 13, D-3508 Melsungen (DE)**

(74) Vertreter: **von Kreisler, Alek, Dipl.-Chem. et al, Deichmannhaus am Hauptbahnhof, D-5000 Köln 1 (DE)**

(54) **Anschlussvorrichtung.**

(57) Eine erfindungsgemäße Anschlußvorrichtung zur Verbindung einer Kanüle mit einem Überleitungsschlauch zur kontinuierlichen Einführung einer Flüssigkeit, insbesondere Insulin, in einen Patienten, ist dadurch gekennzeichnet, daß mit dem patientenfernen Ende der Kanüle in Form eines flexiblen Kurzkatheters (1) ein offener Hohlstutzen (12) verbunden ist, der mit einem axial durchbohrten Ansatz (17) des Überleitungsschlauches (16) lösbar verbindbar ist, daß in dem Hohlstutzen (12) eine durchstechbare Membran (15) aus elastischem Material angeordnet ist und daß an dem Ansatz (17) des Überleitungsschlauches (16) eine Stahlkanüle (19) befestigt ist, die mit ihrem spitzen Ende über die Stirnfläche des Ansatzes (17) um ein Stück vorsteht und die Membran (15) durchsticht. Auf diese Weise wird die Belastung des Patienten bei Abnahme und Anschluß des Überleitungsschlauches vermindert.

## Anschlußvorrichtung

Die Erfindung betrifft eine Anschlußvorrichtung zur
Verbindung einer Kanüle mit einem Überleitungsschlauch
zur kontinuierlichen Einführung einer Flüssigkeit, insbesondere Insulin, in einen Patienten.

Bei der kontinuierlichen Überleitung von Insulin mittels einer Insulinpumpe wird bisher eine Metallkanüle
verwendet, mit deren patientenfernem Ende der Überleitungsschlauch direkt und unlösbar verbunden ist. Mit
der Metallkanüle wird das Gewebe punktiert und sie verbleibt eine Zeitlang in dem Patienten. Zum Abnehmen des
Überleitungsschlauches vom Patienten muß die Metallkanüle aus dem Gewebe herausgezogen werden, was bedeutet,
daß die Stahlkanüle jeden Tag gewechselt werden muß.
Dies ist wegen der jeweils erneuten Punktion eine unerträgliche Belastung für den Patienten.

Der Erfindung liegt die Aufgabe zugrunde, eine Anschlußvorrichtung für ein Überleitungssystem zur kontinuierlichen Gabe von Insulin mittels einer Insulinpumpe zu schaffen, die die Belastung des Patienten bei Abnahme und Anschluß des Überleitungsschlauches vermindert.

Diese Aufgabe wird erfindungsgemäß dadurch gelöst, daß mit dem patientenfernen Ende der Kanüle in Form eines flexiblen Kurzkatheters ein offener Hohlstutzen verbunden ist, der mit einem axial durchbohrten Ansatz des Überleitungsschlauches lösbar verbindbar ist, daß in dem Hohlstutzen eine durchstechbare Membran aus elastischem Material angeordnet ist und daß an dem Ansatz des Überleitungsschlauches eine Stahlkanüle befestigt ist, die mit ihrem spitzen Ende über die Stirnfläche des Ansatzes um ein Stück vorsteht und die Membran durchsticht.

Auf diese Weise ergibt sich eine lösbare Kupplung zwischen Kanüle und Überleitungsschlauch, die eine problemlose Verbindung und Trennung dieser beiden Teile ermöglicht, so daß die flexible Kanüle eine gewisse längere Zeit in dem Patienten verbleiben und der Überleitungsschlauch täglich erneut an den liegenden Kurzkatheter angeschlossen werden kann. Dabei ist besonders vorteilhaft, daß keine Metallkanüle, sondern ein flexibler Kurzkatheter benutzt wird, der das Gewebe schont. Sobald der Ansatz des Überleitungsschlauches mit dem Hohlstutzen an dem Kurzkatheter zusammengesetzt ist, hat die Stahlkanüle des Ansatzes die Membran in dem Hohlstutzen durchstochen und der Insulinfluß in den Patienten ist freigegeben. Bei Trennung des Ansatzes des Überleitungsschlauches von dem Hohlstutzen wird die Stahlkanüle aus der Membran herausgezogen, diese schließt die Einstichöffnung durch Eigenelastizität und bewirkt einen Verschluß des Kurzkatheters, der sowohl das Eindringen von Kontaminationen in den Kurzkatheter als auch den Austritt von Blut oder Gewebeflüssigkeit aus dem Kurzkatheter verhindert. Ein in dieser Weise gesicherter Gewebezugang zum Anschluß des Überleitungsschlauches kann mehrere Tage liegenbleiben und dem Patienten wird die Belastung von sich täglich wiederholenden Punktionen erspart.

In vorteilhafter Ausgestaltung der Erfindung ist vorgesehen, daß der Hohlstutzen Teil eines Kupplungsstückes mit einem axialen Kanal ist und daß die beiden Enden des Kupplungsstückes mit einem Ansatz des Kurzkatheters bzw. mit dem Ansatz des Überleitungsschlauches lösbar verriegelbar zusammensteckbar sind. Hierbei handelt es sich um eine herstellungsmäßig vorteilhafte Alternative zu der Möglichkeit der Verwendung eines bekannten Katheteransatzes selbst als Hohlstutzen, in den die durchstechbare Membran eingebaut und mit dem der Ansatz des Überleitungsschlauches verbindbar ist. Zweckmäßigerweise ist die Membran als Boden eines hutförmigen Elastomerkörpers ausgebildet, der in einen angepaßten Hohlraum des Hohlstutzens eingesetzt ist. Der Boden des hutförmigen Elastomerkörpers ist zweckmäßigerweise dem patientenfernen äußeren Ende des Hohlstutzens zugewandt, so daß - bei Ausbildung des Ansatzes des Überleitungsschlauches als Einsteckteil - die Stirnflächen des Bodens und des Ansatzes in zusammengestecktem Zustand einander berühren und das vorstehende Stück der Stahlkanüle zum Durchbohren der Membran nur unwesentlich länger sein muß als die Dicke der Membran.

In der Innenwand des Hohlstutzens ist mindestens eine axiale Längsprofilierung ausgebildet, die mit mindestens einer axialen Längsprofilierung an der äußeren Umfangsfläche des Ansatzes des Überleitungsschlauches als Drehsicherung zusammengreift. Die Längsprofilierungen sind vorteilhafterweise als Längsnut an dem einen Teil und als Längsrippe an dem anderen Teil gestaltet. Diese Ausbildung verhindert, daß Hohlstutzen und Ansatz des Überleitungsschlauches sich während ihrer gegenseitigen Verbindung relativ zueinander drehen können. Eine solche Drehung hätte nämlich auch eine Drehung der Stahlkanüle beim Durchstechen der Membran und hierdurch bedingten Abrieb von Latex oder Silikonpartikeln der Membran zur Folge, die in den Patienten eingeführt und diesen gefährden würden. Die Drehsicherung ist insbesondere dann wichtig, wenn vorgesehen ist, daß zur Verriegelung des Hohlstutzens mit dem Ansatz des Überleitungsschlauches radiale Verriegelungsnocken an dem einen Teil und ein Gewinde an einer mit dem anderen Teil lose drehbar verbundenen Überwurfmutter angeordnet sind. Vorteilhafterweise ist die lose drehbare Überwurfmutter an dem Ansatz des Überleitungsschlauches angebracht.

Die Überwurfmutter und der Hohlstutzen sind jeweils mittels einer Schutzkappe verschließbar, so daß bei abgekuppeltem Überleitungsschlauch die Enden der beiden Teile gegen Kontamination geschützt sind.

In der Zeichnung ist ein Ausführungsbeispiel der Erfindung dargestellt. Es zeigen:

Fig. 1 einen Längsschnitt einer zusammengebauten Anschlußvorrichtung,

Fig. 2 einen Längsschnitt des verschlossenen Ansatzes des Überleitungsschlauches und

Fig. 3 einen Längsschnitt des verschlossenen Kupplungsstückes mit Kurzkatheter.

Der durch Punktion in einen Patienten einführbare Teil besteht aus einem flexiblen Kurzkatheter 1 (Microcath), mit dem ein hohler Ansatz 2 fest verbunden ist, der einen Luer-Innenkonus 3 und an seiner Außenseite radial abstehende Verriegelungsnocken 4 aufweist. An dem Ansatz 2 sind zur Längsachse des Kurzkatheters 1 quer gerichtete Flügel 5 angebracht, mit deren Hilfe der eingeführte Kurzkatheter 1 auf der Haut des Patienten festgeklebt wird.

Mit dem Kurzkatheter 1 ist ein Kupplungsstück 6 lösbar verbunden. Das Kupplungsstück 6 besteht aus Kunststoff und weist an einem Ende eine Muffe 7 mit Innengewinde 8 auf, die einen Luer-Außenkonus 9 mit radialem Abstand umgibt. Das Innengewinde 8 wirkt mit den Verriegelungsnocken 4 des Ansatzes 2 des Kurzkatheters 1 zusammen und beim Zusammenschrauben der beiden Teile dringt der Außenkonus 9 passend und abdichtend in den Innenkonus 3 des Ansatzes 2 ein. In dem gezeichneten Verbindungszu-stand der Teile ist ein zentraler Kanalabschnitt 10 in dem Außenkonus 9 mit dem Durchlaß des Kurzkatheters 1 verbunden. Der Kanalteil 10 setzt sich durch eine Querwand 11 des Kupplungsstückes 6 hindurch fort und mündet in einen Hohlstutzen 12 des Kupplungsstückes 6. Die Querwand 11 und der Hohlstutzen 12 können einstückig ausgebildet sein. Gegebenenfalls können sie - wie dargestellt - aus zwei durch Ultraschall miteinander verschweißten Teilen bestehen. Der Hohlstutzen 12 weist einen Halsteil 12a auf, der an seinem patientenfernen Mündungsrand nach außen radial abstehende Verriegelungsnocken 13 trägt. Der Rest des Halsteiles 12a ist außen glattwandig. An den Halsteil 12a schließt sich ein Basisteil 12b größeren äußeren Durchmessers an. Der Hohlraum des aus Halsteil 12a und Basisteil 12b bestehenden Hohlstutzens 12 ist einmal abgestuft und seine Form ist dem Außenprofil eines hutförmigen Elastomerkörpers 14 angepaßt, der ein Sackloch enthält, das als Kanalteil 10a den Kanalteil 10 in dem Außenkonus 9 und der Querwand 11 koaxial fortsetzt, wenn der Elastomerkörper 14 in den Hohlraum eingefügt ist. Der Kanalteil 10a endet an dem geschlossenen Boden des Elastomerkörpers 14, der als durchstechbare Membran 15 dient. Der Elastomerkörper 14 kann aus Latex oder Silikon hergestellt sein. Die Stirnfläche seines verbreiterten Basisteils liegt gegen die gerade Fläche der Querwand 11 bündig an und die Abstufung des Hohlraumes des Hohlstutzens 12 hält den eingepaßten Ringteil größeren Durchmessers des Elastomerkörpers 14 in dem Hohlstutzen 12 fest.

Das Kupplungsstück 6 hat die Aufgabe, einen an eine nicht gezeichnete tragbare Insulinpumpe angeschlossenen Überleitungsschlauch 16 mit dem Kurzkatheter 1 lösbar zu verbinden, so daß ein beliebig trennbares Überleitungssystem zur kontinuierlichen Gabe von Insulin entsteht. Der Überleitungsschlauch 16 ist an seinem einen Ende mit einem Ansatz 17 fest verbunden, der als Einsteckteil mit einem Außenkonus 18 ausgestattet ist. Das äußere Ende des Ansatzes 17 enthält eine zu dem Kanal des Überleitungsschlauches 16 koaxiale Bohrung, in die eine Stahlkanüle 19 festsitzend eingelassen ist. Das spitze Ende der Stahlkanüle 19 steht ein Stück über die Stirnfläche des Außenkonus 18 des Ansatzes 17 vor. Das überstehende Stück ist etwas länger als die Dicke des Bodens 15 des Elastomerkörpers 14, so daß die angeschliffene Spitze der Stahlkanüle 19 in den Kanalteil 10a hineinragt, wenn der Außenkonus 18 in die entsprechend konische Mündungsöffnung des Halsteiles 12a des Hohlstutzens 12 eingesteckt ist.

Zur Verriegelung des Ansatzes 17 und des Kupplungsstückes 6 dient eine Überwurfmutter 20, die gegen axiales Abziehen von dem Ansatz 17 gesichert auf diesem
lose drehbar vorgesehen ist. In dem Muffenteil der
Überwurfmutter 20 ist ein Gewinde 21 ausgebildet, das
mit den Verriegelungsnocken 13 des Halsteiles 12a des
Hohlstutzens 12 zusammenwirkt. Um bei der Verbindung
des Ansatzes 17 und des Halsteiles 12a eine Drehung der
beiden Teile relativ zueinander und damit einen Abrieb
von Elastomermaterial des Bodens 15 durch die sich drehende Stahlkanüle 19 zu verhindern, ist in der Innenwand des Halsteiles 12a eine gerade Längsnut 22 ausgebildet, in die eine Längsrippe 27 auf der Außenfläche
des Außenkonus 18 des Ansatzes 17 als Drehsicherung
eingreift.

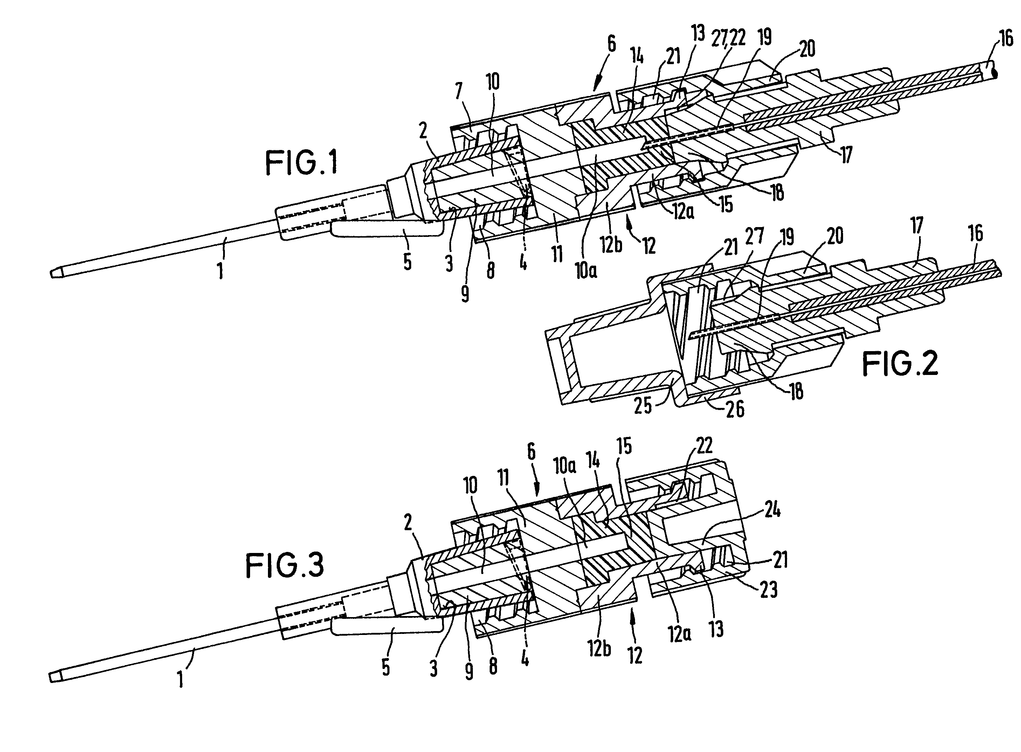

Bei Benutzung der Anschlußvorrichtung sind ihre Bauteile wie in Fig. 1 gezeigt angeordnet. Zur Abkupplung des
Überleitungsschlauches 16 von dem Kurzkatheter 1 wird
die Überwurfmutter 20 von dem Halsteil 12a des Kupplungsstückes 6 abgeschraubt und es wird dabei die
Stahlkanüle 19 aus dem Boden 15 des Elastomerkörpers 14
axial herausgezogen. Durch Eigenelastizität wird das
Einstichloch in dem Boden 15 unverzüglich geschlossen,
so daß sowohl das Austreten von Körperflüssigkeit durch
die Kanalteile 10,10a als auch das Eindringen von Kontaminationen verhindert wird. Als zusätzliche Sicherheit kann auf den Halsteil 12a eine Verschlußkappe 23
(Fig.3) aufgeschraubt werden, deren Luer-Außenkonus 24
in die Mündungsöffnung des Halsteiles 12a eindringt und
dabei den Elastomerkörper 14 etwas staucht, wodurch die
Abdichtung des Durchstichloches verbessert wird. Aus
hygienischen Gründen wird auch das freie Ende des Überleitungsschlauches 16 mit einer Schutzkappe 25 gegen
äußere Einflüsse gesichert. Die Schutzkappe 25 übergreift mit ihrem Kappenteil 26 das offene Ende des Muffenteiles der Überwurfmutter 20, auf den sie passend
aufgesteckt ist. Ein nützlicher Zusatzeffekt der
Schutzkappe 25 besteht darin, daß sie Verletzungsgefahr
durch das überstehende Stück der Stahlkanüle 19 unterbindet.

ANSPRÜCHE

1. Anschlußvorrichtung zur Verbindung einer Kanüle mit einem Überleitungsschlauch zur kontinuierlichen Einführung einer Flüssigkeit, insbesondere Insulin, in einen Patienten,
d a d u r c h   g e k e n n z e i c h n e t,   daß mit dem patientenfernen Ende der Kanüle in Form eines flexiblen Kurzkatheters (1) ein offener Hohlstutzen (12) verbunden ist, der mit einem axial durchbohrten Ansatz (17) des Überleitungsschlauches (16) lösbar verbindbar ist, daß in dem Hohlstutzen (12) eine durchstechbare Membran (15) aus elastischem Material angeordnet ist und daß an dem Ansatz (17) des Überleitungsschlauches (16) eine Stahlkanüle (19) befestigt ist, die mit ihrem spitzen Ende über die Stirnfläche des Ansatzes (17) um ein Stück vorsteht und die Membran (15) durchsticht.

2. Anschlußvorrichtung nach Anspruch 1,
d a d u r c h   g e k e n n z e i c h n e t,   daß der Hohlstutzen (12) Teil eines Kupplungsstückes (6) mit einem axialen Kanal ist und daß die beiden Enden des Kupplungsstückes (6) mit einem Ansatz (2) des Kurzkatheters (1) bzw. mit dem Ansatz (17) des Überleitungsschlauches (16) lösbar verriegelbar zusammensteckbar sind.

3. Anschlußvorrichtung nach Anspruch 2,
d a d u r c h   g e k e n n z e i c h n e t,   daß die Anschlußenden des Kupplungstückes (6) und der beiden Ansätze (2;17) als Innenkonus bzw. Außenkonus gestaltet sind.

4. Anschlußvorrichtung nach einem der Ansprüche 1 - 3, d a d u r c h   g e k e n n z e i c h n e t,   daß die Membran (15) als Boden eines hutförmigen Elastomerkörpers (14) ausgebildet ist, der in einen angepaßten Hohlraum des Hohlstutzens (12) eingesetzt ist.

5. Anschlußvorrichtung nach einem der Ansprüche 1 - 4, d a d u r c h   g e k e n n z e i c h n e t,   daß an der Innenwand des Hohlstutzens (12) mindestens eine axiale Längsprofilierung ausgebildet ist, die mit mindestens einer axialen Längsprofilierung an der äußeren Umfangsfläche des Ansatzes (17) des Überleitungsschlauches (16) als Drehsicherung zusammengreift.

6. Anschlußvorrichtung nach Anspruch 5, d a d u r c h   g e k e n n z e i c h n e t,   daß die Längsprofilierungen als Längsnut (22) an dem einen Teil (6) und als Längsrippe (27) an dem anderen Teil (17) gestaltet sind.

7. Anschlußvorrichtung nach einem der Ansprüche 1 - 6, d a d u r c h   g e k e n n z e i c h n e t,   daß zur Verriegelung des Hohlstutzens (12) mit dem Ansatz (17) des Überleitungsschlauches (16) radiale Verriegelungsnocken (13) an dem einen Teil (12) und ein Gewinde (21) an einer mit dem anderen Teil (17) lose drehbar verbundenen Überwurfmutter (20) angeordnet sind.

8. Anschlußvorrichtung nach einem der Ansprüche 1 - 7, d a d u r c h   g e k e n n z e i c h n e t,   daß die Überwurfmutter (20) und der Hohlstutzen (12) jeweils mittels einer Schutzkappe (23;25) verschließbar sind.

FIG.1

FIG.2

FIG.3

−1/1−

0157224